# EUROPÄISCHE PATENTSCHRIFT

(11) **EP 1 115 343 B1**
(45) Veröffentlichungstag und Bekanntmachung des Hinweises auf die Patenterteilung: **08.12.2004**
(21) Anmeldenummer: 98922698.0
(22) Anmeldetag: 14.04.1998
(51) Int. Cl.: A61B 18/12

(54) **EINRICHTUNG ZUR ÜBERWACHUNG DER APPLIKATION EINER NEUTRALELEKTRODE**
DEVICE FOR MONITORING THE APPLICATION OF A NEUTRAL ELECTRODE
DISPOSITIF POUR SURVEILLER L'APPLICATION D'UNE ELECTRODE NEUTRE

(30) Priorität: 10.04.1997 DE 19714972
(43) Veröffentlichungstag der Anmeldung: 18.07.2001
(73) Patentinhaber: Storz Endoskop GmbH, 8200 Schaffhausen (CH)
(72) Erfinder: DANERS, Felix, CH-8203 Schaffhausen (CH)
(74) Vertreter: Meissner, Bolte & Partner
(86) Internationale Anmeldenummer: PCT/EP1998/002153
(87) Internationale Veröffentlichungsnummer: WO 1998/044855

(56) Entgegenhaltungen:
- EP-A- 0 359 217
- DE-C- 3 239 640
- US-A- 4 651 280
- US-A- 4 741 334
- US-A- 4 754 757
- US-A- 5 406 503

## Beschreibung

Die Erfindung bezieht sich auf eine Einrichtung zur Überwachung der Applikation einer zweiteiligen Neutralelectrode bei der monopolaren HF-Chirurgie.

Bei solchen Einrichtungen besteht dann, wenn die tatsäcliliclne Kontaktfläche der Neutralelektrode mit dem Körper des Patienten zu klein ist, aufgrund der dann auftretenden großen Stromdichten die Gefahr von Verbrennungen.

### Stand der Technik

Einrichtungen zur Überwachung der Applikation von Neutralelektroden in der Hochfrequenzchirurgie sind aus der DE 32 39 640 C2 oder der EP 0 390 937 A1 bekannt. Die DE 32 39 640 C2 offenbart eine solche Einrichtung mit einem Impedanzsensor, der einen Resonanzkreis mit einer Sekundärspule eines Transformators und HF-Einkoppelkondensatoren vorzugsweise zweier Elektrodenteilflächen aufweist, der in Serie geschaltete Übergangsimpedanzen zweier Elektrodenteilflächen zum Patientengewebe durch Anlegen eines Patientenhilfsstromes erfaßt und durch die Übergangsimpedanzen bedämpft ist.

Wenn dieses Resonanznetzwerk genau mit seiner Resonanzfrequenz angeregt wird, wird der Patientenübergangswiderstand R allein sichtbar. Dieser Arbeitspunkt läßt sich im Prinzip durch eine Spannungsmitkopplung erreichen. Es zeigt sich jedoch, daß solche Anordnungen anfällig auf die von HF-Generatoren erzeugten Störsignale reagieren. Eine Spannungsmitkopplung müßte nach einem Filter abgegriffen werden, welches selbst je nach Bauteiltoleranz zu Phasenfehlern führt. Der Parallelresonanzkreis wird dann nicht mehr genau bei seiner Resonanzfrequenz angeregt, was zu Meßfehlern führt. Ferner wird ein solches System unübersichtlich aufgrund der Initialtoleranz vieler beteiligter Bauteile.

### Darstellung der Erfindung

Es ist daher Aufgabe der Erfindung eine Einrichtung zur monopolaren HF-Chirurgie bereitzustellen, die den Resonanzkreis in einer Weise anregt, die eine fehlerfreie Messung der Übergangsimpedanz zum Patientengewebe ermöglicht.

Eine erfindungsgemäße Lösung dieser Aufgabe ist im Patentanspruch 1 angegeben. Weiterbildungen der Erfindung sind Gegenstand der Ansprüche 2 bis 20.

Gemäß Anspruch 1 wird die Aufgabe dadurch gelöst, daß der Resonanzkreis mit Wechselspannungen variierender Frequenzen im Bereich einer Resonanzfrequenz angeregt wird und daß ein Spitzenwertdetektor vorgesehen ist, der einen bei der Resonanzfrequenz auftretenden Spitzenwert der Wechselspannung erfaßt.

Im Gegensatz zu herkömmlichen Meßeinrichtungen, die aufgrund von Bauteiltoleranzen mit Phasenfehlern behaftet sind, verzichtet die erfindungsgemäße Einrichtung auf die Verwendung jeglicher Rückkopplungssignale, die durch die großen Störsignale verändert werden könnten. Statt dessen ermittelt der Spitzenwertdetektor den bei der Resonanzfrequenz auftretenden Spitzenwert der Wechselspannung. Während die Frequenz der anregenden Spannung fortlaufend verändert, in der Regel in Form eines Frequenz-sweeps wiederholt durchfahren wird, speichert der Spitzenwertdetektor, dessen Zeitkonstante wesentlich größer als die Dauer einer einzelnen Frequenzvariation oder eines einzelnen Sweeps ist, den erfaßten Spitzenwert. Dieser entspricht dem Patientenübergangswiderstand bei der Resonanzfrequenz, die selbst erfindungsgemäß überhaupt nicht bestimmt wird. Durch die Auswahl des während der Frequenzvariation erfaßten Spitzenwerts als Patientenübergangswiderstand werden Meßfehler herkömmlicher Vorrichtungen, die die Resonanzfrequenz genau zu treffen versuchten, vermieden.

Bevorzugte Ausführungsformen der Unteransprüche 2 bis 5 werden im Rahmen der Figurenbeschreibung näher erläutert.

Damit die Funktion des Impedanzsensors im Selbsttest, d.h. ohne HF-Generator geprüft werden kann, sieht eine Weiterbildung der Erfindung gemäß Anspruch 6 einen Referenzresonanzkreis vor, der über einen Schalter anstelle des Resonanzkreises angeschlossen werden kann. Der Referenzwiderstand ist entsprechend Anspruch 7 bei der Resonanzfrequenz vorteilhafterweise größer als der höchste Patientenübergangswiderstand, der noch eine Aktivierung der HF-Generatorenleistung erlaubt. Dadurch ist in Verbindung mit den Ausführungsformen der Ansprüche 7 und 8 sichergestellt, daß bei einem Versagen des Schalters beim Zurückschalten auf das Resonanznetzwerk keine HF-Anwendung möglich ist.

Die aus den genannten Druckschriften bekannten Anordnungen beziehungsweise Einrichtungen zur Überwachung der Applikation von Neutralelektroden erzeugen ein Signal, wenn die Applikation der Neutralelektrode so schlecht geworden ist, daß der HF-Generator nicht mehr aktiviert werden darf. Bei eingeschaltetem HF-Generator wird aufgrund des zu hohen Übergangswiderstandes der Neutralelektrode plötzlich die HF-Ausgangsleistung abgeschaltet.

Herkömmliche Einrichtungen sind daher nicht geeignet, den Chirurgen auf das Schlechterwerden der Applikation der Neutralelektrode hinzuweisen oder bereits beim Anlegen der Neutralelektrode eine Aussage über die Qualität der Applikation zu liefern. Wünschenswert ist daher eine kontinuierliche Überwachung der Applikation sowie eine Feststellung der Güte der Applikation bereits beim Anlegen der Neutralelektrode.

Die in Anspruch 10 vorgeschlagene Weiterbildung der Erfindung bietet daher die Möglichkeit, während des Anlegens der Neutralelektrode schon festzustellen, wie gut sie plaziert ist. Während der Operation kann zudem festgestellt werden, ob die Stromdichte ansteigt und bald einen kritischen Wert erreichen wird, bei dem der Generator nicht mehr aktiviert werden kann. Darüber hinaus erlaubt diese Weiterbildung festzustellen, ob die Neutralelektrode aufgrund ihrer physikalischen Eigenschaften, ihrer Ausdehnung und ihrer Plazierung für den geplanten Eingriff geeignet oder zu knapp bemessen ist.

Ausgehend von einem System zur monopolaren HF-Chirurgie mit einer einfachen, geteilten Neutralelektrode kann als Parameter zur Beschreibung der Güte der Applikation außer dem Übergangswiderstand auch die Differenz der Ströme, die durch die beiden Teile der Neutralelektrode fließen, in an sich bekannter Weise ausgewertet werden. Dementsprechend weisen Weiterbildungen gemäß Anspruch 10 ff außer dem Impedanzsensor, der die in Serie geschalteten Übergangswiderstände der beiden Elektrodenteilflächen durch Anlegen eines Patientenhilfsstromes erfaßt, noch einen Stromasymmetriesensor auf.

Der Stromasymmetriesensor erfaßt bei aktiviertem HF-Ausgangssignal die Differenz zwischen den durch die beiden Elektrodenteilflächen fließenden HF-Ströme und gemäß Anspruch 15 bei nicht aktiviertem HF-Ausgangssignal den Gleichrichtwert des Patientenhilfsstroms zwischen den beiden Elektrodenteilflächen. Die Ausgangssignale des Impedanzsensors und gemäß Anspruch 13 auch des Stromasymmetriesensors sind an eine Steuerund Auswerteelektronik angelegt, die gemäß Anspruch 18 aus den Signalen des Impedanzsensors und des Stromasymmetriesensors ein Signal zur Überwachung der Sensorfunktion ableitet. Hierzu kann die Auswerte- und Steuereinheit gemäß Anspruch 16 neben dem momentanen Übergangswiderstand auch die momentane Größe der Stromasymmetrie im Verhältnis zu einem HF-Strom sowie die erste Ableitung dieser relativen Stromasymmetrie nach der Zeit als Kriterien benutzen.

Es ist ferner zweckmäßig, daß die Steuer- und Auswer-Leelektronik die Ausgangssignale des Impedanzsensors und des Stromasymmetriesensors in Bezug zur gewählten Generatoreinstellung setzt. Für die Beurteilungs- und Verknüpfungsvorgänge kann dabei neben fest vorgegebenen Kriterien für die Übergangsimpedanz und die Asymmetrie gemäß Anspruch 19 eine Fuzzy-Logic-Funktion eingesetzt werden, die die einzelnen Parameter miteinander verknüpft.

Die erfindungsgemäße Einrichtung kann schließlich gemäß Anspruch 20 eine optische und/oder akustische Anzeigeeinrichtung, wie beispielsweise eine Balkenanzeige aufweisen, die die Güte der Applikation der Neutralelektrode anzeigt und den Chirurgen vor einem Ausfall der Neutralelektrode warnt.

### Kurze Beschreibung der Zeichnung

Die Erfindung wird nachstehend ohne Beschränkung des allgemeinen Erfindungsgedankens anhand eines Ausführungsbeispieles unter Bezugnahme auf die Zeichnungen exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: den prinzipiellen Aufbau des Impedanzsensors,
- Fig. 2: den Aufbau des Resonanznetzwerkes und der HF-Einkoppelung,
- Fig. 3: den Aufbau des Bandpaß-Filters, des Spitzenwertdetektors und des Meßsignalverstärkers, und
- Fig. 4: den Aufbau des Stromasymmetriesensors.

### Darstellung von Ausführungsbeispielen

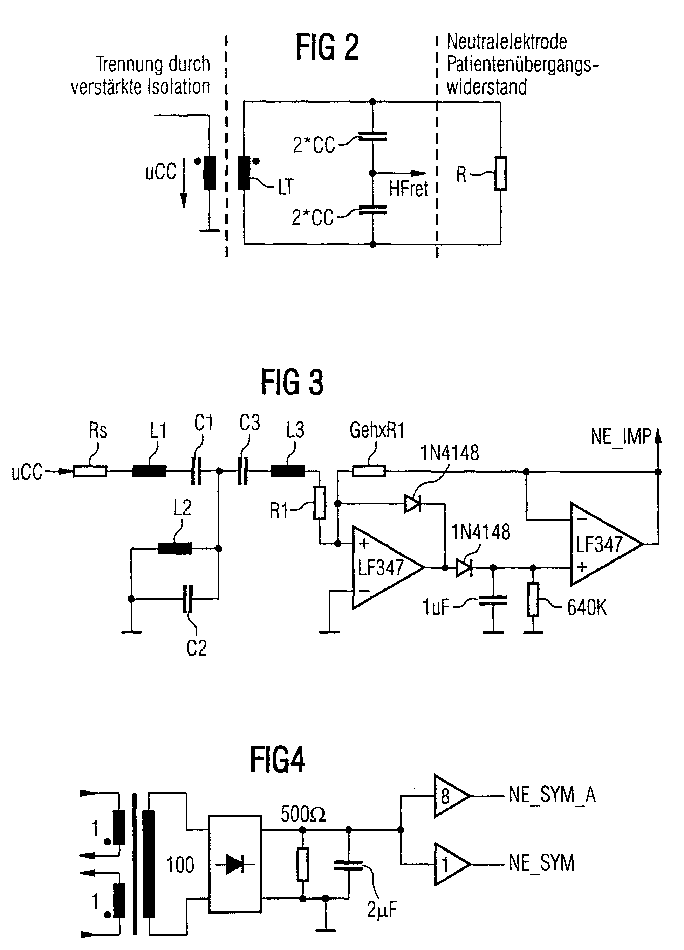

Bei der Realisierung einer Impedanzüberwachung der Neutralelektrode NE sind die großen Störsignale bei aktiviertem HF-Generator problematisch. Damit der HF-Strom symmetrisch auf die Elektrodenteilflächen eingespeist werden kann, wird - wie Fig. 2 zeigt - üblicherweise jede Teilfläche der Neutralelektrode über einen Kondensator 2*CC an den gleichen Pol HFret des HF-Generators angeschlossen.

Die Einrichtung zur Erfassung der Patientenübergangsimpedanz bzw. des Patentenübergangswiderstandes ist von den Elektrodenteilflächen galvanisch getrennt. Zur galvanischen Trennung wird ein Transformator verwendet, dessen Sekundärwicklung LT zwischen die Elektrodenteilflächen geschaltet wird; die Primärseite liegt auf dem Potential der Steuer- und Auswerteelektronik.

Die Sekundärspule des zur Widerstandstransformation dienenden Transformators bildet mit den Einkoppelkondensatoren 2*CC eine Parallelresonanz, die durch den Patientenübergangswiderstand R bedämpft wird.

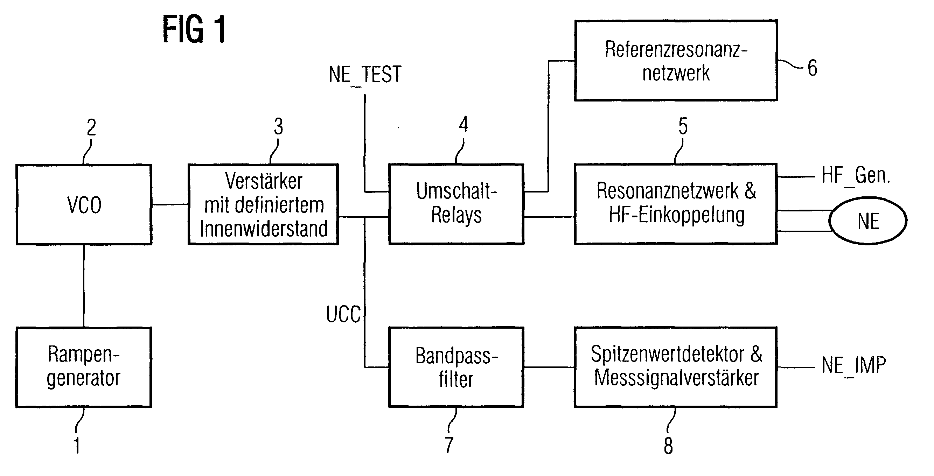

Das Resonanznetzwerk, d.h. die Parallelresonanz aus der Transformatorhauptreaktanz LT und den Einkoppelkondensatoren 2*CC, wird mit einem Frequenzsweep, d.h. mit variierenden Frequenzen angeregt. Hierzu dient gemäß Figur 1 ein VCO 2, das von einem Rampengenerator 1 angesteuert wird, und dessen Ausgangssignal an einem Verstärker 3 mit definiertem Innenwiderstand anliegt.

Der VCO 2 muß den gesamten Parallelresonanzfrequenzbereich überstreichen, damit die Resonanzfrequenz gefunden wird. Bevorzugt besteht der VCO 2 aus einer Schaltung, die keinen Frequenzabgleich erfordert.

Der Rampengenerator 1 wird aus zwei Komparatoren aufgebaut, damit die Minimal- und Maximalspannung der Spannungsrampe genau eingestellt werden kann.

Der Rampengenerator 1 hat typischerweise eine Anstiegsund Abfallzeit von ca. 6ms. Die Anstiegszeit entspricht gleichzeitig der Abtastrate, mit der der Patientenübergangswiderstand zwischen den Elektrodenteilflächen gemessen wird.

Das Ausgangssignal des Verstärkers 3 ist über ein Umschaltrelais 4 entweder an dem Resonanznetzwerk 5, das in Fig. 2 dargestellt ist, oder an einem Referenzresonanznetzwerk 6 angelegt, das an späterer Stelle erläutert wird.

Bei der Beaufschlagung der Resonanznetzwerke 5 bzw. 6 ist die Eingangsimpedanz des jeweiligen Netzwerkes immer genau bei der Resonanzfrequenz am größten und entspricht dem Patientenübergangswiderstand R zwischen den Elektrodenteilflächen. Da das jeweilige Resonanznetzwerk durch eine Spannungsquelle mit definiertem Innenwiderstand, nämlich den Verstärker 3 angeregt wird, liegt immer genau bei der Resonanzfrequenz die größte Spannungsamplitude an der Parallelresonanz an.

Zur Bestimmung der Resonanzfrequenz werden der Bandpaßfilter 7 und der Spitzwertdetektor und Meßsignalverstärker 8 verwendet, deren Aufbau in Fig. 3 im einzelnen dargestellt ist.

Das Bandpaßfilter 7 hat einen Durchlaßbereich großer Bandbreite und bietet eine gute Dämpfung gegen Störsignale. Die Bandbreite des Durchlaßbereichs berücksichtigt beispielsweise Initialtoleranzen verwendeter Bauteile, Verschiebungen aufgrund von Temperaturänderungen oder Alterungsprozessen sowie einen Sicherheitsabstand, so daß die Resonanzfrequenz in jedem Fall erfaßt wird. Da erfindungsgemäß nicht die Phase, sondern die größte Spannungsamplitude ausgewertet wird, bestehen keine Anforderungen an den Phasenverlauf.

Die von dem Verstärker 3 erzeugte HF-Spannung UCC, die auch an der Parallelresonanz anliegt, wird über das Bandpaßfilter 7 an den Spitzenwertdetektor 8 angelegt und von diesem gemessen. Die Zeitkonstante des Detektors ist viel größer als die Zeit, die ein Frequenzsweep benötigt. Dadurch liegt am Detektorausgangsanschluß eine Spannung NE_IMP an, die praktisch nur vom Patientenübergangswiderstand R zwischen den Elektrodenteilflächen, dem Innenwiderstand und der Spannung der anregenden Spannungsquelle 3 abhängt. Die Spannung NE_IMP kann dann von einer geeigneten, nicht dargestellten Auswerteelektronik ausgewertet werden.

Damit die Funktion des Impedanzsensors im Selbsttest, d.h. ohne HF-Generator geprüft werden kann, ist ein Referenzresonanznetzwerk 6 vorhanden, das durch das Umschaltrelais 4 anstelle des Resonanznetzwerkes 5 auf die Sensorelektronik geschaltet werden kann. Das Referenzresonanznetzwerk wird aus einem Parallelresonanzkreis aus einer kleinen Festinduktivität, einem Kondensator und einem Referenzwiderstand aufgebaut. Die Induktivität und die Kapazität entspricht ungefähr den Werten des Resonanznetzwerkes. Der Referenzwiderstand ist größer als der höchste Patientenübergangswiderstand, der noch eine Aktivierung der HF-Generatorleistung erlaubt. Dadurch ist sichergestellt, daß bei einem Versagen des Umschaltrelais 4 beim Zurückschalten auf das Resonanznetzwerk keine Aktivierung möglich ist.

Die Sensorausgangsspannung NE_IMP muß also in einem genau vorgegebenen Spannungsfenster liegen, wenn der Impedanzsensor auf das Resonanznetzwerk geschaltet ist.
Bei fehlerhaftem Verhalten kann mit dem gleichen Umschalr-relais der Patientenhilfsstrom abgeschaltet werden. NE_TEST bezeichnet das Eingangssignal, das das Umschaltrelais 4 steuert und insbesondere zwischen Selbsttest und Überwachung des Patientenübergangswiderstandes bei nicht aktiviertem HF-Generator umschaltet.

Als geeignetes Arbeitsfrequenzband bietet sich 40-80 kHz an. In jedem Fall wird die Bandbreite ausreichend groß gewählt, um die Resonanzfrequenz auch angesichts Initialtoleranzen der verwendeten Bauteile oder Verschiebungen durch Temperatureinflüsse oder Alterungsprozesse sicher zu erfassen. Der Abstand zur HF-Generatorarbeitsfrequenz ist genügend groß; weiterhin erlaubt diese Arbeitsfrequenz einen wesentlich größeren Patientenhilfsstrom, der die Messung der kleinen Patientenübergangswiderstände zwischen den Elektrodenteilflächen vereinfacht.

Damit die Störspannungen am Impedanzsensor bei aktiviertem HF-Generator nicht zu groß werden, müssen ausreichend große Einkoppelkondensatoren verwendet werden.

Als Bandpaß wird bei dem in Fig. 3 dargestellten Filter wird eine Butterworthkonfiguration 6. Ordnung benutzt. Sie weist gegenüber der Grundschwingung des HF-Generators eine Dämpfung von etwa 38dB auf.

Das Filter stellt ein T-Glied mit einer Serienresonanz L1, C1, Rs am Eingangsanschluß, einer Parallelresonanz C2, L2 in der Mitte und einer zweiten Serienresonanz am Ausgangsanschluß L3, C3, R1 dar. Die Filterausgangsspannung wird dem Spitzenwertdetektor zugeführt, der gleichzeitig um den Faktor Gain verstärkt.

Für die Dimensionierung des Rampengenerators muß die Einschwingzeit des Filters berücksichtigt werden. Sie ist in etwa um einen Faktor 5 kleiner als die Einschwingzeit des Resonanznetzwerks.

Fig. 4 zeigt den Stromasymmetriesensor. Die Stromasymmetrie in den beiden Elektrodenteilflächen wird am einfachsten mit Hilfe eines Kompensationstransformators KT mit einer Meßwicklung 100 gemessen.

Die Leitungen 1 der Neutralelektrodenteilflächen werden gekreuzt durch die Zentrumsbohrung des Transformators KT gesteckt. Damit heben sich die in gleicher Richtung fließenden Ströme auf. Das resultierende Stromsignal auf der Meßwicklung entspricht gerade dem transformierten asymmetrischen Strom in den Elektrodenteilflächen.

Damit das Signal umabhängig von der Polarität der Generatorspannung bleibt, wird es mit einem Brückengleichrichter gleichgerichtet. Nach dem Brückengleichrichter ist der Abschlußwiderstand angebracht. Der zu messende Maximalwert des Stromes beträgt 1.6 A. Dadurch ist der Abschlußwiderstand mit 500 Ω bestimmt, damit die Sensorspannung beim Maximalstrom 8 V beträgt. Die Spannung an diesem Abschlußwiderstand wird mit einem Kondensator geglättet. Damit stellt die Spannung am Kondensator den Gleichrichtwert des Differenzstromes dar.

Damit der kleine Patientenhilfsstrom im Standby Modus ausgewertet werden kann, wird die Spannung über dem Kondensator durch einen weiteren Verstärker um den Faktor 8 verstärkt. Die Ausgangssignale NE_SYM_A bzw. NE_SYM liegen wiederum an der nicht dargestellten Auswerteeinheit an.

## Patentansprüche

1. Einrichtung zur Überwachung der Applikation einer Neutralelektrode bei der monopolaren HF-Chirurgie mit einem Impedanzsensor, der einen Resonanzkreis (5) mit einer Sekundärspule eines Transformators und HF-Einkoppelkondensatoren vorzugsweise zweier Elektrodenteilflächen aufweist, der in Serie geschaltete Übergangsimpedanzen zweier Elektrodenteilflächen zum Patientengewebe durch Anlegen eines Patientenhilfsstromes erfaßt und durch die Übergangsimpedanzen bedämpft ist,
**dadurch gekennzeichnet, daß** der Resonanzkreis mit Wechselspannungen variierender Frequenzen im Bereich einer Resonanzfrequenz angeregt wird und daß ein Spitzenwertdetektor (8) vorgesehen ist, der einen bei der Resonanzfrequenz auftretenden Spitzenwert der Wechselspannung erfaßt.

2. Einrichtung nach Anspruch 1,
**dadurch gekennzeichnet, daß** der Resonanzkreis durch eine Stromquelle mit definiertem Innenwiderstand angeregt wird.

3. Einrichtung nach Anspruch 1,
**dadurch gekennzeichnet, daß** der Resonanzkreis durch eine Spannungsquelle (1, 2, 3) mit definiertem Innenwiderstand angeregt wird.

4. Einrichtung nach Anspruch 1 oder 3,
**dadurch gekennzeichnet, daß** die Spannungsquelle einen Rampengenerator (1) und ein VCO (2) (voltage controlled oscillator) aufweist.

5. Einrichtung nach Anspruch 3,
**gekennzeichnet durch** einen Bandpaßfilter (7) mit einem Durchlaßbereich, der mindestens einen Toleranzbereich der Resonanzfrequenz überdeckt.

6. Einrichtung einem der Ansprüche 1 bis 5,
**gekennzeichnet durch** ein Referenz-Resonanznetzwerk (6), das während einer Deaktivierung eines HF-Ausgangssignals zur Durchführung eines Selbsttests über einen Schalter (4) anstelle der Neutralelektrode und der Sekundärwicklung des Transformators mit der Spannungsquelle verbunden ist.

7. Einrichtung nach Anspruch 6,
**dadurch gekennzeichnet, daß** das Referenz-Resonanznetzwerk bei der Resonanzfrequenz eine Impedanz besitzt, die größer als der Maximalwert der Übergangsimpedanz ist.

8. Einrichtung einem der Ansprüche 1 bis 7,
**gekennzeichnet durch** eine Steuer- und Auswerteelektronik, die den Verlauf der Übergangsimpedanz auswertet.

9. Einrichtung nach Anspruch 8,
**dadurch gekennzeichnet, daß** die Steuer- und Auswerteelektronik bei Überschreiten eines Maximalwerts der Übergangsimpedanz eine HF-Spannung abschaltet.

10. Einrichtung nach Anspruch 9,
**dadurch gekennzeichnet, daß** die Steuer- und Auswerteelektronik anzeigt, ob eine durch das Patientengewebe fließende Stromdichte ansteigt und sich einem kritischen Wert nähert, bei dem die HF-Spannung abgeschaltet wird.

11. Einrichtung nach einem der Ansprüche 1 bis 10,
**gekennzeichnet durch** einen Stromasymmetriesensor, der während einer Aktivierung des HF-Ausgangssignals eine Differenz **durch** die Elektrodenteilflächen fließender HF-Ströme erfaßt.

12. Einrichtung nach Anspruch 11,
**dadurch gekennzeichnet, daß** der Stromasymmetriesensor einen Kompensationstransformator aufweist.

13. Einrichtung nach Anspruch 12,
**dadurch gekennzeichnet, daß** eine Sekundärspule des Kompensationstransformators zwischen die Elektrodenteilflächen geschaltet ist und eine Primärspule des Stromasymmetriesensors mit der Steuer- und Auswerteelektronik verbunden ist.

14. Einrichtung nach einem der Ansprüche 11 bis 13,
**dadurch gekennzeichnet, daß** Zuleitungen der Elektrodenteilflächen gegensinnig durch eine zentrale Bohrung des Kompensationstransformators geführt sind, so daß die Stromasymmetrie direkt meßbar ist.

15. Einrichtung nach einem der Ansprüche 11 bis 14,
**dadurch gekennzeichnet, daß** der Stromasymmetriesensor während der Deaktivierung des HF-Ausgangssignals den Gleichrichtwert des Patientenhilfsstroms erfaßt.

16. Einrichtung nach einem der Ansprüche 9 bis 15,
**dadurch gekennzeichnet, daß** die Steuer- und Auswerteelektronik das Verhältnis der Stromasymmetrie zu einem HF-Strom und die zeitliche Änderung dieses Verhältnisses auswertet.

17. Einrichtung nach Anspruch 16,
**dadurch gekennzeichnet, daß** die Steuer- und Auswerteelektronik das Signal des Stromasymmetriesensors gleichrichtet und verstärkt.

18. Einrichtung nach einem der Ansprüche 9 bis 17,
gekennzeichnet, daß die Steuer- und Auswerteelektronik aus Signalen des Impedanzsensors und des Stromasymmetriesensors ein Signal für die Güte der Applikation der Neutralelektrode ableitet.

19. Einrichtung nach Anspruch 18,
**dadurch gekennzeichnet, daß** die Steuer- und Auswerteelektronik eine Fuzzy-Logic-Funktion zur Verknüpfung der Signale des Impedanzsensors und des Stromasymmetriesensors mit Parametern der Einrichtung zur HF-Chirurgie aufweist.

20. Einrichtung nach Anspruch 18,
**dadurch gekennzeichnet, daß** die Steuer- und Auswerteelektronik eine optische und/oder akustische Anzeigeeinheit aufweist, die anzeigt, wie hoch die Stromdichte ist und ob sie ansteigt und bald einen kritischen Wert erreichen wird, bei dem der Generator der HF-Spannung nicht mehr aktiviert werden kann.

## Claims

1. A device to monitor the application of a neutral electrode during monopolar RF surgery with an impedance sensor which comprises a resonant circuit (5) with a secondary transformer coil and RF coupling condensers preferably with two surfaces, which record serially connected transition impedances of two electrode surfaces to the patient tissue by applying a patient auxiliary current, and which are dampened by the transition impedances, **characterized in that** the resonant circuit is activated by alternating voltage of varying frequencies in the range of a resonant frequency and that a peak reading detector (8) is provided, which records a peak reading of the alternating voltage occurring in the resonance frequency.

2. The device according to Claim 1, **characterized in that** the resonant circuit is activated by means of a power source with a defined internal resistance.

3. The device according to Claim 1,**characterized in that** the resonant circuit is activated by means of a voltage source (1, 2, 3) with a defined internal resistance.

4. The device according to Claim 1 or 3,**characterized in that** the voltage source has a ramp generator (1) and a VCO (2) (voltage controlled oscillator).

5. The device according to Claim 3, **characterized by** a band-pass filter (7) with a pass band which covers at least one range of tolerance of the resonant frequency.

6. The device according to any one of Claims 1 to 5, **characterized by** a reference resonant circuit (6) which, during deactivation of an RF output signal for the purposes of carrying out an automatic test, is connected to the voltage source via a switch (4) instead of the neutral electrode and the secondary coil of the transformer.

7. The device according to Claim 6, **characterized in that**, at the resonant frequency, the reference resonant circuit has an impedance greater than the peak value of the transition impedance.

8. The device according to any one of Claims 1 to 7, **characterized by** control and evaluation electronics which evaluate the progress of the transition impedance.

9. The device according to Claim 8, **characterized in that** the control and evaluation electronics switch off an RF voltage when a transition impedance peak value is exceeded.

10. The device according to Claim 9, **characterized in that** the control and evaluation electronics indicate whether the current density flowing through the patient tissue is rising and approaching a critical value at which the RF voltage is switched off.

11. The device according to any one of Claims 1 to 10, **characterized by** an asymmetrical voltage sensor which records the difference in RF currents flowing through the electrode surfaces when the RF output signal is activated.

12. The device according to Claim 11, **characterized in that** the asymmetrical voltage sensor has a compensation transformer.

13. The device according to Claim 12, **characterized in that** a secondary coil of the compensation transformer is connected between the electrode surfaces, and a primary coil of the asymmetrical voltage sensor is connected to the control and evaluation electronics.

14. The device according to any one of Claims 11 to 13, **characterized in that** leads run in reverse directions from the electrode surfaces through a central hole in the compensation transformer, so that the voltage asymmetry can be directly measured.

15. The device according to any one of Claims 11 to 14, **characterized in that** the voltage asymmetry sensor records the rectifier value of the patient auxiliary current when the RF output signal is de-activated.

16. The device according to any one of Claims 9 to 15, **characterized in that** the control and evaluation electronics evaluate the relationship of voltage asymmetry to an RF current and the changes in this relationship over time.

17. The device according to Claim 16, **characterized in that** the control and evaluation electronics rectify and amplify the voltage asymmetry sensor signal.

18. The device according to any one of Claims 9 to 17, **characterized in that** the control and evaluation electronics use signals from the impedance sensor and the voltage asymmetry sensor to derive a signal for the quality of the application of the neutral electrode.

19. The device according to Claim 18, **characterized in that** the control and evaluation electronics have a fuzzy logic function to link the signals from the impedance sensor and the voltage asymmetry sensor to parameters of the RF surgery device.

20. The device according to Claim 18, **characterized in that** the control and evaluation electronics have an optical and/or acoustic display unit which indicates how high the current density is, and whether it is rising and will soon reach a critical value at which the RF voltage generator can no longer be activated.

## Revendications

1. Dispositif pour surveiller l'application d'une électrode neutre en chirurgie HF unipolaire, avec un capteur d'impédance présentant un circuit résonant (5) avec un enroulement secondaire d'un transformateur et des condensateurs de couplage HF de préférence de deux parties de surfaces d'électrodes, lequel capteur détecte des impédances transitoires reliées en série de deux parties de surfaces d'électrodes par rapport au tissu du patient résultant de l'application d'un courant auxiliaire au patient et est amorti par les impédances transitoires, **caractérisé en ce que** le circuit résonant est excité avec des tensions alternatives de fréquences variables dans la plage d'une fréquence de résonance et **en ce qu'**il est prévu un détecteur de valeur crête (8) qui détecte la valeur crête de la tension alternative produite à la fréquence de résonance.

2. Dispositif selon la revendication 1, **caractérisé en ce que** le circuit résonant est excité par une source de courant ayant une résistance interne définie.

3. Dispositif selon la revendication 1, **caractérisé en ce que** le circuit résonant est excité par une source de tension (1, 2, 3) ayant une résistance interne définie.

4. Dispositif selon la revendication 1 ou 3, **caractérisé en ce que** la source de tension présente un générateur de rampe (1) et un oscillateur commandé en tension (2) (VCO, *voltage controlled oscillator*).

5. Dispositif selon la revendication 3, **caractérisé par** un filtre passe-bande (7) qui laisse passer une bande de fréquence recouvrant au moins une plage de tolérance de la fréquence de résonance.

6. Dispositif selon l'une des revendications 1 à 5, **caractérisé par** un réseau résonant de référence (6) qui, pendant une désactivation d'un signal de sortie HF, est relié par l'intermédiaire d'un interrupteur (4) à la source de tension, au lieu de l'électrode neutre et de l'enroulement secondaire du transformateur, afin de réaliser un auto-test.

7. Dispositif selon la revendication 6, **caractérisé en ce que**, à la fréquence de résonance, le réseau résonant de référence possède une impédance qui est supérieure à la valeur maximale de l'impédance transitoire.

8. Dispositif selon l'une des revendications 1 à 7, **caractérisé par** une électronique de commande et de traitement du signal qui détermine l'allure de l'impédance transitoire.

9. Dispositif selon la revendication 8, **caractérisé en ce que** l'électronique de commande et de traitement du signal déconnecte une tension HF en cas de dépassement d'une valeur maximale de l'impédance transitoire.

10. Dispositif selon la revendication 9, **caractérisé en ce que** l'électronique de commande et de traitement du signal indique si la densité de courant qui s'écoule dans le tissu du patient augmente et s'approche d'une valeur critique à laquelle la tension HF est déconnectée.

11. Dispositif selon l'une des revendications 1 à 10, **caractérisé par** un capteur d'asymétrie du courant qui, pendant une activation du signal de sortie HF, détecte une différence entre les courants HF qui s'écoulent dans les parties de surfaces d'électrodes.

12. Dispositif selon la revendication 11, **caractérisé en ce que** le capteur d'asymétrie du courant présente un transformateur de compensation.

13. Dispositif selon la revendication 12, **caractérisé en ce qu'**un enroulement secondaire du transformateur de compensation est connecté entre les parties de surfaces d'électrodes et **en ce qu'**un enroulement primaire du capteur d'asymétrie du courant est relié à l'électronique de commande et de traitement du signal.

14. Dispositif selon l'une des revendications 11 à 13, **caractérisé en ce que** des conducteurs de raccordement des parties de surfaces d'électrodes sont guidés en sens contraire dans un perçage central du transformateur de compensation de façon à pouvoir mesurer directement l'asymétrie du courant.

15. Dispositif selon l'une des revendications 11 à 14, **caractérisé en ce que**, pendant la désactivation du signal de sortie HF, le capteur d'asymétrie du courant détecte la valeur moyenne linéaire dans le temps du courant auxiliaire appliqué au patient.

16. Dispositif selon l'une des revendications 9 à 15, **caractérisé en ce que** l'électronique de commande et de traitement du signal détermine le rapport de l'asymétrie du courant par rapport à un courant HF ainsi que la variation de ce rapport dans le temps.

17. Dispositif selon la revendication 16, **caractérisé en ce que** l'électronique de commande et de traitement du signal redresse et amplifie le signal provenant du capteur d'asymétrie du courant.

18. Dispositif selon l'une des revendications 9 à 17, **caractérisé en ce que** l'électronique de commande et de traitement du signal dérive à partir des signaux du capteur d'impédance et du capteur d'asymétrie du courant un signal de qualité de l'application de l'électrode neutre.

19. Dispositif selon la revendication 18, **caractérisé en ce que** l'électronique de commande et de traitement du signal présente une fonction de logique floue pour combiner les signaux du capteur d'impédance et du capteur d'asymétrie du courant avec des paramètres du dispositif de chirurgie HF.

20. Dispositif selon la revendication 18, **caractérisé en ce que** l'électronique de commande et de traitement du signal présente un module de signalisation optique et/ou acoustique qui indique la valeur de la densité de courant et si cette valeur augmente et va bientôt atteindre une valeur critique à laquelle le générateur de tension HF ne pourra plus être activé.
